(19) Europäisches Patentamt
European Patent Office
Office européen des brevets

(11) EP 3 403 653 A1

(12) **EUROPEAN PATENT APPLICATION**

(43) Date of publication:
**21.11.2018 Bulletin 2018/47**

(21) Application number: **17305553.4**

(22) Date of filing: **15.05.2017**

(51) Int Cl.:
***A61K 31/4965*** *(2006.01)*
***A61K 31/155*** *(2006.01)*
***A61K 31/402*** *(2006.01)*
***A61K 31/4709*** *(2006.01)*
***A61K 31/496*** *(2006.01)*
***A61K 31/55*** *(2006.01)*
***A61P 25/18*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicants:
- **Centre National de la Recherche Scientifique (CNRS)**
  **75016 Paris (FR)**
- **Université De Nice Sophia Antipolis**
  **06103 Nice (FR)**

(72) Inventors:
- **COUNILLON, Laurent**
  **06100 NICE (FR)**
- **MILOSAVLJEVIC, Nina**
  **MANCHESTER, M1 3BR (GB)**
- **POET, MALLORIE**
  **06100 NICE (FR)**

(74) Representative: **Cabinet Plasseraud**
**235 Cours Lafayette**
**69006 Lyon (FR)**

(54) **COMPOUNDS FOR USE IN TREATING BIPOLAR AND RELATED DISORDERS**

(57) The present invention relates to compounds for use in treating bipolar and related disorders.

EP 3 403 653 A1

## Description

### FIELD OF THE INVENTION

The present invention relates to compounds for use in treating bipolar and related disorders.

### BACKGROUND OF THE INVENTION

Bipolar disorder is characterized by recurrent episodes of mania or elevated mood, and irritable or depressive moods, which proved to lead psychosocial disability, especially when left untreated.

Currently, the main treatment of bipolar disorder is a long term treatment which includes, *inter alia,* the use of valproic acid, lamotrigine, carbamazepine and lithium in the form of salts such as lithium carbonate or lithium citrate.

To date, lithium is considered to be the most effective treatment although it suffers from many downsides. Indeed, the side effects of a treatment based on lithium include leucocytose, polyurea, polydypsia, migraine, memory loss, mental confusion, muscle weakness, nausea, vomittin, vertigo and electrocardiography changes and kidney damage, in addition to interfering with glucose metabolism. These limitations render the use of lithium and its salt inacceptable for a long term treatment.

In addition, in some dramatic cases, it has been documented that a misuse or overdose of lithium may induce coma and eventually death. In addition, the first manifestations resulting from an overdose of lithium are usually similar to the side effects conferred by the treatment *per se,* and therefore may be not accurately acknowledged by the patient.

The currently used medicines thus fail to provide for an appropriate treatment. There is a long-time felt unfulfilled need for an effective new therapeutic strategy for specifically targeting bipolar disorder, especially with reduced secondary effects.

### SUMMARY OF THE INVENTION

The inventors have put in light, for the very first time, a mechanism underlying the action of lithium. The inventors have indeed shown that lithium act as an inhibitor of the specific Na+/H+ exchanger NHE7.

The inventors further investigated new therapeutic compounds that would alleviate the side effects of lithium, while maintaining a high efficiency in the treatment of bipolar patients. They have shown that derivatives of guanidine inhibit NHE7, which indicates highly promising effects in the treatment of bipolar and related disorder.

Consequently, **in a first aspect,** the invention relates to a compound for use in treating bipolar and related disorders, wherein said compound is represented by formula (I):

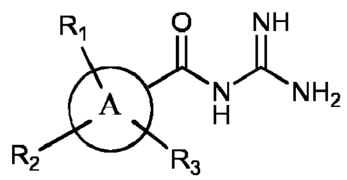

(I)

wherein :

- A is a cycle selected from the group consisting of phenyl and heteroaryl having 5 or 6 ring atoms;
- $R_1$ is selected from the group consisting of $C_1$-$C_6$ alkyl, $C_3$-$C_6$ cycloalkyl, $C_1$-$C_6$ haloalkyl, halogen, $C_1$-$C_6$ alkylsulfonyl, $C_1$-$C_6$ alkylsulfoxide, heteroaryl having 5 to 10 ring atoms, aryl having 6 to 10 ring atoms, heterocycle having 5 to 7 ring atoms, -$NR_4R_5$, -$OR_6$, nitro, cyano, sulfonate, and aminosulfonyl ;
    - $R_4$, $R_5$ and $R_6$ being independently chosen among hydrogen and $C_1$-$C_6$ alkyl;
- $R_2$ is selected from the group consisting of $C_1$-$C_6$ alkyl, -$NR_4R_5$, -$OR_6$, $C_7$-$C_{16}$ arylalkyl, $C_7$-$C_{16}$ alkylaryl, $C_1$-$C_6$ alkenyl, and $C_1$-$C_6$ alkynyl, heteroaryl having 5 to 10 ring atoms, aryl having 6 to 10 ring atoms and heterocycle having 5 to 7 ring atoms, said heterocycle being optionally substituted by - (C=O)-$R_7$;
    - $R_4$, $R_5$ and $R_6$ being as defined above,
    - $R_7$ being chosen among heteroaryl having 5 to 10 ring atoms and aryl having 6 to 10 ring atoms;
- $R_3$ can be present or absent and, if present, $R_3$ is selected from the group consisting of $C_1$-$C_6$ alkyl, and -$NR_4R_5$,

- $R_4$ and $R_5$ being as defined above;

and wherein any alkyl, cycloalkyl, haloalkyl, alkylsulfonyl, alkylsulfoxide, heteroaryl, aryl, heterocycle, arylalkyl, alkylaryl, alkenyl or alkynyl group is optionally substituted with one or more substituents selected from the group consisting of $C_1$-$C_6$ alkyl, halogen, $C_1$-$C_6$ haloalkyl, oxo, nitro, cyano, $-NH_2$, -OH, - SH, -COOH, and $-CONH_2$.

**In a second aspect,** the invention relates to a composition comprising at least one compound of formula (I), and at least one further agent selected from the group consisting:

- mood stabilizers such as lithium or its salts including lithium carbonate, lithium citrate, lithium sulfate, lithium orotate, and lithium oxybutyrate;
- anticonvulsants such as valproic acid, divalproex sodium, lamotrigine, carbamazepine, gabapentin, topiramate and oxcarbazepine; and
- atypical antipsychotics such as olanzapine, aripiprazole, quetiapine, risperidone, ziprasidone, asenapine, paliperidone, and lurasidone

**In a third aspect,** the invention relates to a pharmaceutical composition comprising at least one compound of formula (I), and at least one further agent as disclosed herein.

**In a fourth aspect,** the invention relates to said pharmaceutical composition for use in therapy, preferably for treating bipolar and related disorders.

**In a fifth aspect,** the invention relates to at least one compound of formula (I), and at least one further agent as a combined preparation for simultaneous, separate or sequential use for treating bipolar or related disorders.

## DETAILED DESCRIPTION OF THE INVENTION

### ***Compound for use in a method for treating bipolar disorder***

Although widely used for treating bipolar disorder, the scientific community did not clearly elucidate the mechanism underlying the action of lithium.

The inventors have now deciphered, for the very first time, a biological pathway explaining the lithium key role in the treatment of bipolar disorder. Indeed, they have shown that lithium acts as a partial agonist of intracellular $Na^+/H^+$ exchanger NHE7, a $Na^+/H^+$ exchanger massively expressed in the brain. Being an agonist of NHE7, lithium thus challenges sodium towards NHE7. This results in the inhibition of NHE7.

Consequently, the inventors have put in light that intracellular $Na^+/H^+$ exchanger NHE7 is inhibited by lithium and plays a critical role in the neuroplasticity and cerebral integration functions.

Starting from this unexpected and groundbreaking discovery of the mechanism explaining the effects mediated by lithium, the inventors met the burden to show that a therapeutic class of compounds provide an efficient effect in the treatment of patients suffering from bipolar disorder and related disorders.

Indeed, the inventors have shown that derivatives of guanidine inhibit NHE7, which indicates highly promising effects in the treatment of bipolar and related disorder.

Thus, **in a first aspect,** the invention relates to a compound for use for treating bipolar and related disorders, wherein said compound is represented by formula (I):

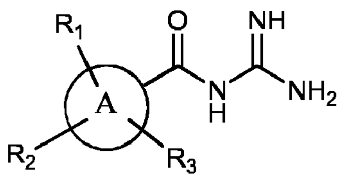

(I)

wherein :

- A is a cycle selected from the group consisting of phenyl and heteroaryl having 5 or 6 ring atoms;
- $R_1$ is selected from the group consisting of $C_1$-$C_6$ alkyl, $C_3$-$C_6$ cycloalkyl, $C_1$-$C_6$ haloalkyl, halogen, $C_1$-$C_6$ alkylsulfonyl, $C_1$-$C_6$ alkylsulfoxide, heteroaryl having 5 to 10 ring atoms, aryl having 6 to 10 ring atoms, heterocycle having 5 to 7 ring atoms, $-NR_4R_5$, $-OR_6$, nitro, cyano, sulfonate, and aminosulfonyl ;
    - $R_4$, $R_5$ and $R_6$ being independently chosen among hydrogen and $C_1$-$C_6$ alkyl;

- $R_2$ is selected from the group consisting of $C_1$-$C_6$ alkyl, $-NR_4R_5$, $-OR_6$, $C_7$-$C_{16}$ arylalkyl, $C_7$-$C_{16}$ alkylaryl, $C_1$-$C_6$ alkenyl, and $C_1$-$C_6$ alkynyl, heteroaryl having 5 to 10 ring atoms, aryl having 6 to 10 ring atoms and heterocycle having 5 to 7 ring atoms, said heterocycle being optionally substituted by - (C=O)-$R_7$;
  - $R_4$, $R_5$ and $R_6$ being as defined above,
  - $R_7$ being chosen among heteroaryl having 5 to 10 ring atoms and aryl having 6 to 10 ring atoms;
- $R_3$ can be present or absent and, if present, $R_3$ is selected from the group consisting of $C_1$-$C_6$ alkyl, and $-NR_4R_5$,
  - $R_4$ and $R_5$ being as defined above;

and wherein any alkyl, cycloalkyl, haloalkyl, alkylsulfonyl, alkylsulfoxide, heteroaryl, aryl, heterocycle, arylalkyl, alkylaryl, alkenyl or alkynyl group is optionally substituted with one or more substituents selected from the group consisting of $C_1$-$C_6$ alkyl, halogen, $C_1$-$C_6$ haloalkyl, oxo, nitro, cyano, $-NH_2$, -OH, - SH, -COOH, and $-CONH_2$.

The invention also encompasses the compound of formula (I), tautomers, enantiomers, diastereomers, racemates or mixtures thereof, or hydrates, solvates, pharmaceutically acceptable salts for use in treating bipolar and related disorders.

The terms **"$C_1$-$C_6$ alkyl"**, by itself or as part of another substituent, refer to a linear or branched alkyl functional group having 1 to 6 carbon atoms. Suitable alkyl groups include methyl, ethyl, *n*-propyl, *i*-propyl, *n*-butyl, *i*-butyl, *s*-butyl and *t*-butyl, pentyl and its isomers (e.g. *n*-pentyl, *iso*-pentyl), and hexyl and its isomers (e.g. *n*-hexyl, *iso*-hexyl).

The terms **"$C_3$-$C_6$ cycloalkyl"** refer to a cyclic hydrocarbyl radical having 3 to 6 carbon atoms. Suitable cycloalkyl groups include cyclopropyl, cyclobutyl, cyclopentyl and cyclohexyl.

The term **"halogen"** refers to a fluoro (-F), chloro (-Cl), bromo (-Br), or iodo (-I) group.

The terms **"$C_1$-$C_6$ haloalkyl"** refer to a $C_1$-$C_6$ alkyl as defined above wherein one or more hydrogen atoms on the alkyl are replaced by a halogen group as defined above. Suitable $C_1$-$C_6$ haloalkyl groups include trifluoromethyl.

The terms **"$C_1$-$C_6$ alkylsulfonyl"** refer to a $-SO_2$-R group, wherein R is a $C_1$-$C_6$ alkyl as defined above.

The terms **"$C_1$-$C_6$ alkylsulfoxide"** refer to a -SO-R group, wherein R is a $C_1$-$C_6$ alkyl as defined above.

The terms **"aryl having 6 to 10 ring atoms"** refer to a polyunsaturated, aromatic hydrocarbyl group having a single ring (i.e. phenyl) or multiple aromatic rings fused together (e.g. naphtyl), containing 6 to 10 atoms, wherein at least one ring is aromatic. The aromatic ring may optionally include one to two additional rings (cycloalkyl, heterocyclyl or heteroaryl) fused thereto. Suitable aryl groups include phenyl and naphtyl.

The terms **"heteroaryl having 5 to 10 ring atoms"** refer to aromatic rings or ring systems containing 1 to 2 rings which are fused together or linked covalently, containing 5 to 10 atoms; at least one of which is aromatic, in which one or more carbon atoms in one or more of these rings is replaced by oxygen, nitrogen and/or sulfur atoms where the nitrogen and sulfur heteroatoms may optionally be oxidized and the nitrogen heteroatoms may optionally be quaternized. Such rings may be fused to an aryl, cycloalkyl, heteroaryl or heterocyclyl ring. Non-limiting examples of such heteroaryl, include: furanyl, thiophenyl, pyrrolyl, pyrazolyl, imidazolyl, oxazolyl, isoxazolyl, thiazolyl, isothiazolyl, triazolyl, oxadiazolyl, thiadiazolyl, tetrazolyl, oxatriazolyl, thiatriazolyl, pyridinyl, pyrimidyl, pyrazinyl, pyridazinyl, oxazinyl, dioxinyl, thiazinyl, triazinyl, indolyl, isoindolyl, benzofuranyl, isobenzofuranyl, benzothiophenyl, isobenzothiophenyl, indazolyl, benzimidazolyl, benzoxazolyl, purinyl, benzodioxolyl, benzodioxanyl, benzothiadiazolyl, quinolinyl, isoquinolinyl, cinnolinyl, quinazolinyl and quinoxalinyl.

The terms **"heteroaryl having 5 to 6 ring atoms"** refer to an aromatic ring containing 5 to 6 atoms; in which one or more carbon atoms is replaced by oxygen, nitrogen and/or sulfur atoms where the nitrogen and sulfur heteroatoms may optionally be oxidized and the nitrogen heteroatoms may optionally be quaternized. Non-limiting examples of such heteroaryl, include: furanyl, thiophenyl, pyrrolyl, pyrazolyl, imidazolyl, oxazolyl, isoxazolyl, thiazolyl, isothiazolyl, triazolyl, oxadiazolyl, thiadiazolyl, tetrazolyl, oxatriazolyl, thiatriazolyl, pyridinyl, pyrimidyl, pyrazinyl, pyridazinyl, oxazinyl, dioxinyl, thiazinyl, triazinyl.

The terms **"heterocycle having 5 to 7 ring atoms"** refer to a cyclic compound having as ring members, atoms of at least two different elements, like carbon and oxygen atoms, or carbon and nitrogen atoms, or carbon and sulfur atoms. Examples of heterocycle include, but are not limited to, 1-(1,2,5,6-tetrahydropyridyl), 1-piperidinyl, 2-piperidinyl, 3-piperidinyl, 4-morpholinyl, 3-morpholinyl, tetrahydrofuran-2-yl, tetrahydrofuran-3-yl, tetrahydrothien-2-yl, tetrahydrothien-3-yl, 1-piperazinyl, 2-piperazinyl, 1-azepanyl, and the like.

The terms **"$C_7$-$C_{16}$ arylalkyl"** refer to an aryl-alkyl- moiety, wherein the aryl has 6 to 10 ring atoms as defined above, and the alkyl is a $C_1$-$C_6$ alkyl as defined above. Arylalkyl groups include, for example, benzyl groups.

The terms **"$C_7$-$C_{16}$ alkylaryl"** refer to an alkyl-aryl- moiety, wherein the aryl has 6 to 10 ring atoms as defined above, and the alkyl is a $C_1$-$C_6$ alkyl as defined above.

The terms **"$C_1$-$C_6$ alkenyl"** refer to a straight or branched hydrocarbon moiety having at least one carbon-carbon double bond. Alkenyl groups include, for example, ethenyl (i.e., vinyl), propenyl, butenyl, 1-methyl-2-buten-1-yl,

pentenyl, hexenyl, octenyl, and butadienyl.

The terms **"$C_1$-$C_6$ alkynyl"** refer to a straight or branched hydrocarbon moiety having at least one carbon-carbon triple bond. Examples of "alkynyl" include ethynyl, 2-propynyl (propargyl), 1-propynyl, pentynyl, hexynyl, and allenyl groups, and the like.

In one embodiment:

- A is a cycle selected from the group consisting of phenyl and heteroaryl having 5 or 6 ring atoms;
- $R_1$ is selected from the group consisting of $C_1$-$C_6$ alkyl, $C_3$-$C_6$ cycloalkyl, $C_1$-$C_6$ haloalkyl, halogen, $C_1$-$C_6$ alkylsulfonyl, heteroaryl having 5 to 10 ring atoms, aryl having 6 to 10 ring atoms, heterocycle having 5 to 7 ring atoms, and $-NR_4R_5$;
    - $R_4$ and $R_5$ being independently chosen among hydrogen and $C_1$-$C_6$ alkyl;
- $R_2$ is selected from the group consisting of $C_1$-$C_6$ alkyl, $-NR_4R_5$, heteroaryl having 5 to 10 ring atoms, aryl having 6 to 10 ring atoms, and heterocycle having 5 to 7 ring atoms, said heterocycle being optionally substituted by -(C=O)-$R_7$,;
    - $R_4$ and $R_5$ being as defined above,
    - $R_7$ being chosen among heteroaryl having 5 to 10 ring atoms and aryl having 6 to 10 ring atoms;
- $R_3$ can be present or absent and, if present, $R_3$ is selected from the group consisting of $C_1$-$C_6$ alkyl, and $-NR_4R_5$,
    - $R_4$ and $R_5$ being as defined above.

Typically, A is selected from the group consisting of phenyl, pyrazine and pyrazole. In one embodiment, A is selected from the group consisting of

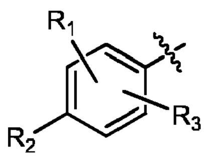, 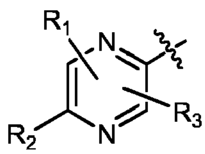, 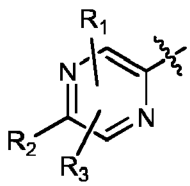, and 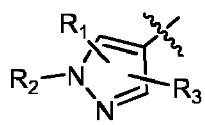.

According to a preferred embodiment, A is selected from the group consisting of

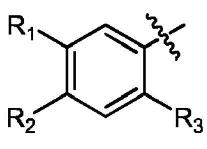, 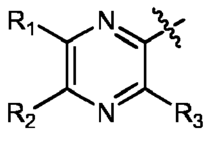, 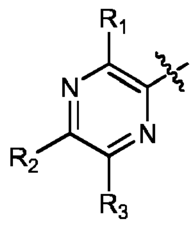, and 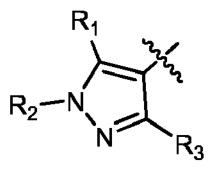.

In one embodiment:

- $R_1$ is selected from the group consisting of $C_3$-$C_6$ cycloalkyl, halogen, $C_1$-$C_6$ alkylsulfonyl, and $C_1$-$C_6$ haloalkyl;
- $R_2$ is selected from the group consisting of heteroaryl having 5 to 10 ring atoms and aryl having 6 to 10 ring atoms;
- $R_3$ can be present or absent and, if present, $R_3$ is a $C_1$-$C_6$ alkyl group.

In one embodiment, $R_1$ is a halogen, for example -Cl. In another embodiment, $R_1$ is a $C_1$-$C_6$ alkylsulfonyl, for example methylsulfonyl. In another embodiment, $R_1$ is a $C_1$-$C_6$ haloalkyl, for example trifluoromethyl. In another embodiment, $R_1$ is a $C_3$-$C_6$ cycloalkyl, for example cyclopropyl. In another embodiment, $R_1$ represents $-NR_4R_5$. In this specific embodiment, $R_4$ and $R_5$ are independently chosen among hydrogen and $C_1$-$C_6$ alkyl group. Said alkyl group may be a linear chain alkyl group or a branched chain alkyl group.

In one embodiment, $R_2$ represents $-NR_4R_5$. In this specific embodiment, $R_4$ and $R_5$ are independently chosen among hydrogen and $C_1$-$C_6$ alkyl group. Said alkyl group may be a linear chain alkyl group, like methyl or ethyl, or a

branched chain alkyl group, like *i*-propyl. In another embodiment, $R_2$ represents a $C_1$-$C_6$ alkyl, for example a branched alkyl group like *i*-propyl. In another embodiment, $R_2$ represents a heteroaryl, for example pyrrole or quinolinyl. In another embodiment, $R_2$ represents a heterocycle substituted by -(C=O)-$R_7$, for example a piperazine substituted by -(C=O)-$R_7$, $R_7$ being a pyrrolyl.

In one embodiment, $R_3$ represents -$NR_4R_5$. In this specific embodiment, $R_4$ and $R_5$ are independently chosen among hydrogen and $C_1$-$C_6$ alkyl group. Said alkyl group may be a linear chain alkyl group or a branched chain alkyl group. In another embodiment, $R_3$ represents a $C_1$-$C_6$ alkyl, for example methyl.

Thus, in one embodiment, the invention relates to a compound for use for treating bipolar and related disorders, wherein said compound is represented by formula (I):

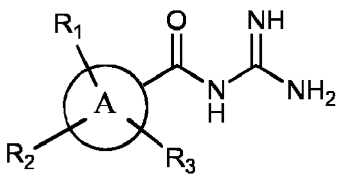

(I)

wherein :

- A is a cycle selected from the group consisting of phenyl, pyrazine and pyrazole;
- $R_1$ is selected from the group consisting of cyclopropyl, Cl, methylsulfonyl, trifluoromethyl, and -$NH_2$,
- $R_2$ is selected from the group consisting of isopropyl, -$NH_2$, dimethylamino, ethyl-*i*-propylamino, 1-piperazinyl substituted by -(C=O)-$R_7$, 1-azepanyl, pyrrolyl, and quinolinyl,
    - $R_7$ being a pyrrolyl;
- $R_3$ can be present or absent and, if present, $R_3$ is selected from the group consisting of methyl and -$NH_2$.

According to one embodiment, the compound for use for treating bipolar and related disorders is selected in the group consisting of the compounds as represented by formula (II), formula (III), formula (IV), formula (V), formula (VI), formula (VII), formula (VIII), and formula (IX):

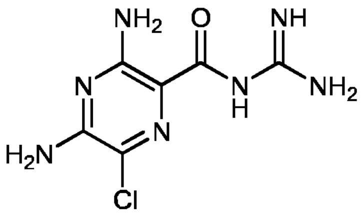

(II) ;

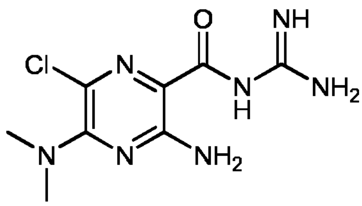

(III)

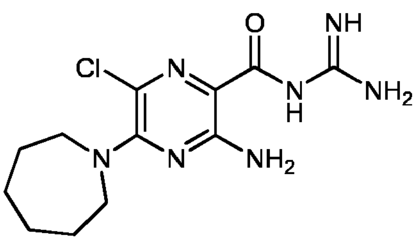

(IV) ;

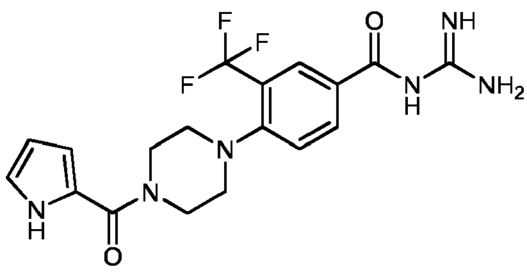

(V)

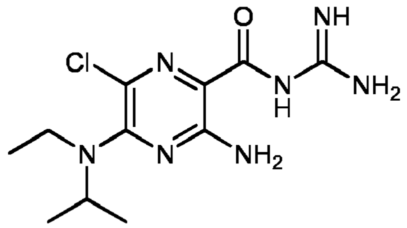

(VI);

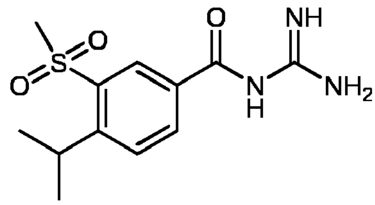

(VII)

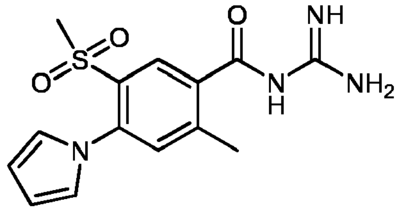

(VIII);

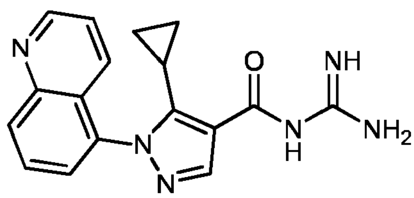

(IX).

According to a preferred embodiment, the compound for use for treating bipolar and related disorders is selected in the group consisting of the compounds as represented by formula (VIII) and formula (IX).

Thus, in one embodiment, the compound of the invention is represented by formula (II):

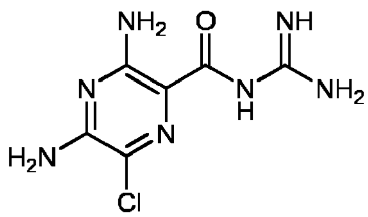

(II)

The compound of formula (II) is called amiloride.

In one embodiment, the compound of the invention is represented by formula (III):

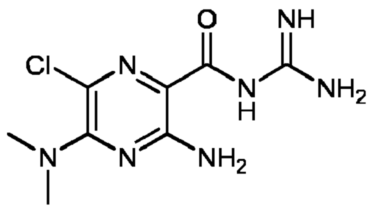

(III)

The compound of formula (II) is called dimethyl-amiloride also referred to as DMA.

In one embodiment, the compound of the invention is represented by formula (IV):

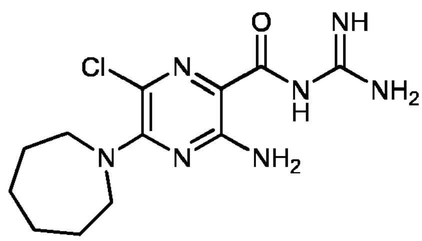

(IV)

The compound of formula (IV) is called hexamethyl-amiloride, also referred to as HMA.

In one embodiment, the compound of the invention is represented by formula (V):

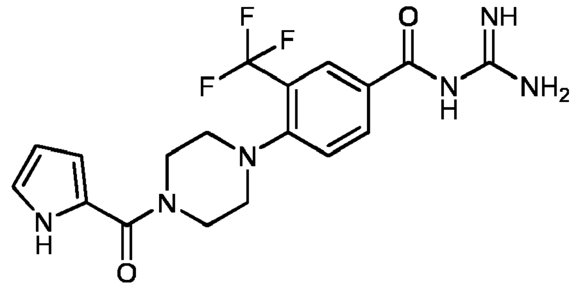

(V)

The compound of formula (V) is called sabiporide.

In one embodiment, the compound of the invention is represented by formula (VI):

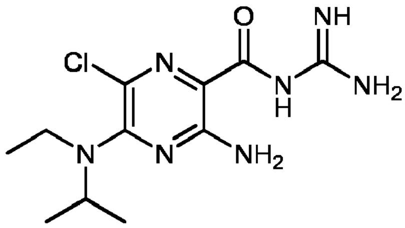

(VI)

The compound of formula (VI) is called 5N-(ethylisopropyl) amiloride, also referred to as EIPA.

In one embodiment, the compound of the invention is represented by formula (VII):

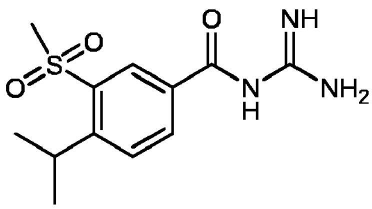

(VII)

The compound of formula (VII) is called cariporide.

In one embodiment, the compound of the invention is represented by formula (VIII):

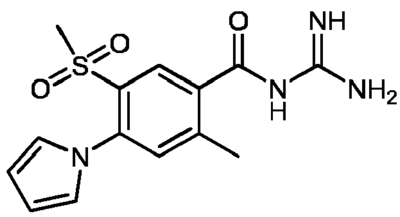

(VIII)

The compound of formula (VIII) is called eniporide.

In one embodiment, the compound of the invention is represented by formula (IX):

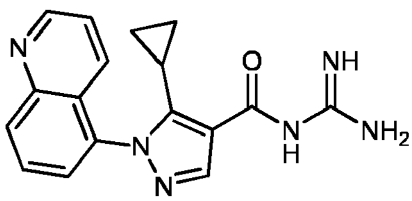

(IX)

The compound of formula (IX) is called zoniporide.

As used herein, the terms **"compound of formula (I)"** or **"compound of the invention"** are meant to include any compounds that falls within the definition of formula (I), as well as their salts, solvates, tautomers and stereoisomers. The agents of the invention are intended to be used for treating bipolar disorders. Said compounds falls within the definition of acylgunanidines.

Preferably, the compound of the invention is able to go through the blood-brain barrier. For achieving this purpose, the compound is preferably hydrophobic.

As previously mentioned, the inventors have shown that the compound of the invention acts as inhibitors of NHE7. As used herein, **"NHE7"** or **"sodium hydrogen exchanger 7"** refers to an intracellular exchanger. Said exchanger is responsible for the acidification of early endosomal compartments. The inventors have shown that said exchanger is expressed in large integrative neurons such as Purkinje cells in the cerebellum, pyramidal cells in hippocampus and cortex, or motor neurons in brainstem.

Thus, NHE7 acts as a proton leading transport rather than a proton leak. NHE7 mediates an acidification of intracellular vesicles that is additive to that of V-ATPases and that accelerates endocytosis.

By inhibiting NHE7, the compounds of the invention would achieve the exact same effect than lithium on this transporter and on endocytosis. Therefore, in one embodiment, the invention relates to the compound of the invention for use for treating bipolar and related disorders by inhibiting NHE7. In one embodiment, the compound of the invention selectively inhibits NHE7 compare to NHE1. The term "selectively" refers to a difference of Ki by a factor 10, 100 or more between NHE7 and NHE1. It has to be added that as NHE7 has a much lower Km for sodium than NHE1, and as the described compounds are competitive inhibitors, they are much more potent on NHE7 than on NHE1.

The compounds belonging to the acylguanidine family are widely known in therapy, especially as antiarrhythmic medicine. The available clinical data have shown that said compounds are non-toxic. The inventors have now shown an unforeseen mechanism mediated by said compounds. Said newly uncovered mechanism explains the promising effect of the compounds of the invention on bipolar patients. Therefore, the compounds of the invention address the problem of treating bipolar disorder, but would not shown the same limitation than the currently widely used lithium.

As used herein, **"bipolar disorder"** refers to a psychiatric illness which pathophysiology still remains poorly understood. However, the disease is thoroughly disclosed in :

- the "Diagnostic and Statistical Manual of Mental Disorders, Fifth Edition, also referred to as **"DSM-V".** DSM the 2013 update to the American Psychiatric Association's classification and diagnostic tool
- the "International Statistical Classification of Diseases and Related Health Problems", usually called by the short-form name International Classification of Diseases (ICD). ICD is the international standard diagnostic tool for epidemiology, health management and clinical purposes and is maintained by the World Health Organization. The current version is **"ICD-10".**

It is noteworthy that ICD10 refers to bipolar disorder as "bipolar affective disorder" and is defined as including several spectrum of the disease. Consequently, **"bipolar and related disorders"** include but are not limited to :

- Bipolar affective disorder, current episode hypomanic;
- Bipolar affective disorder, current episode manic without psychotic symptoms;
- Bipolar affective disorder, current episode manic with psychotic symptoms;
- Bipolar affective disorder, current episode mild or moderate depression;
- Bipolar affective disorder, current episode severe depression without psychotic symptoms ;
- Bipolar affective disorder, current episode severe depression with psychotic symptoms ;
- Bipolar affective disorder, current episode mixed ;
- Bipolar affective disorder, currently in remission ;
- Other bipolar affective disorders including bipolar II disorder; and
- Unspecified Bipolar affective disorder.

As for the DSM-V, the bipolar disorder has been subdivided into bipolar I, bipolar II, cyclothymia, based on the nature and severity of mood episodes experienced. Said subtypes are thus meant to be included in the definition of "bipolar and related disorders" according to the present invention.

Bipolar I disorder is qualified by at least one lifetime manic or mixed episode. Mania must last at least one week or require hospitalization and symptoms during this period include irritability, euphoria, decreased need for sleep, grandiose ideas, impulsive behavior, increased talkativeness, racing thoughts, increased activity and distractibility.

Bipolar II disorder consists of periods of hypomania and major depression. Hypomania is similar to mania but considered milder in that the patient is not socially or vocationally impaired but shows changes in functioning.

Cyclothymic disorder relates to a chronic state of cycling between hypomanic and depressive episodes that do not reach the diagnostic standard for bipolar disorder

Despite ICD10 and DSMV define the bipolar disorder in their own way, it is acknowledged that **"bipolar disorder"** is a psychiatric diagnosis that describes a category of mood disorders defined by the presence of episode(s) of abnormally elevated mood clinically referred to as mania or, if milder, hypomania, and episode(s) of depression. The mood change is usually accompanied by a change in the overall level of activity; most of the other symptoms are either secondary to, or easily understood in the context of, the change in mood and activity.

Individuals who experience manic episodes also commonly experience depressive episodes or symptoms, or mixed episodes in which features of both mania and depression are present at the same time. These episodes are usually separated by periods of "normal" mood, but in some individuals, depression and mania may rapidly alternate, known as rapid cycling.

Extreme manic episodes can sometimes lead to psychotic symptoms such as delusions and hallucinations. It is noteworthy that bipolar disorder represents the modern understanding of the classic manic-depressive disorder described in the nineteenth century. Therefore, the very definition of the disease evolved over the year. This evolution illustrates the development of our own comprehension of the disease.

Thus, the modern comprehension of this disorder differs from the classic description in that psychotic symptoms or psychosis as such is not a requirement. Put in other words, a patient suffering from bipolar disorder does not necessarily suffer from psychotic symptoms.

As used herein, **"psychotic symptoms"** refers to delusions, hallucinations, confused and disturbed thoughts, lack of insight and self-awareness. Preferably, said expression refers to delusions and hallucinations. Said symptoms are experienced by patients whose link to reality is impaired. Basically, people experiencing psychotic breaks are totally unaware that their delusions or hallucinations are not real.

Consequently, in the sense of the invention, bipolar disorder preferably refers to a disorder which does not include psychosis or any psychotic symptoms. The treated subjects do thus not show any signs of psychosis or psychotic symptoms.

Consequently, bipolar disorder, as meant in the present invention preferably excludes schizoaffective disorder.

As used herein, the term **"treating"** or **"treatment",** denotes reversing, alleviating, inhibiting the progress of, or preventing the disorder or condition to which such term applies, or reversing, alleviating, inhibiting the progress of, or preventing one or more symptoms of the disorder or condition to which such term applies. Treatment of bipolar disorder is thus aimed at treating the manic, hypomanic and depressive symptoms of the disorder as well as at maintaining treatment by reducing or preventing the cyclicity of the disorder. Consequently, in another aspect, this invention also relates to the compounds of the invention for use for diminishing, inhibiting or eliminating the symptoms associated with bipolar disorder including

- manic episodes which is characterized by unrealistic plans, recklessness, decreased need for sleep, pressured speech, inflated self-esteem, uncharacteristically poor judgment, indifferent to personal grooming, and unusual sexual drive, and
- depressive episodes, which is characterized by manifestation of sadness, hypersomnolence, insomnia, anorexia, bulimia, feeling of loneliness, apathy or indifference, depersonalization, loss of interest in sexual activity, shyness, social anxiety, lack of motivation, morbid/suicidal ideation, irritability, and restlessness.

## ***Combination of the compounds of the invention and a further agent and uses thereof***

The inventors further investigated the newly unveiled effect of the compound of the invention. They came to the conclusion that said compound show better results when in combination with a further agent. Thus, **in a second aspect,** the invention relates to a composition comprising at least one compound of formula (I) as described above, and at least one further agent, wherein said further agent is selected from the group consisting:

- mood stabilizers such as lithium or its salts including lithium carbonate, lithium citrate, lithium sulfate, lithium orotate, and lithium oxybutyrate;
- anticonvulsants such as valproic acid, divalproex sodium, lamotrigine, carbamazepine, gabapentin, topiramate and oxcarbazepine; and
- atypical antipsychotics such as olanzapine, aripiprazole, quetiapine, risperidone, ziprasidone, asenapine, paliperidone, and lurasidone.

The composition of the invention provides unexpected and novel perspective for therapeutic intervention on patients suffering from bipolar disorder or related disorder. The treatment with a combination of:

- a compound of the invention, such as amiloride, dimethyl-amiloride, hexamethyl amilordie, sabiporide, 5N-(ethylisopropyl) amiloride, cariporide, eniporide or zoniporide ; and

- a further agent aimed to treat bipolar disorder,

proved to be highly useful for treating bipolar or related disorders.

As used herein, the expression **"further agent"** refers to an agent which is commonly used for treating patients suffering from bipolar disorder. As previously mentioned said agent include, but is not limited to mood stabilizer, anticonvulsant or atypical antispychotics. Preferably, said further agent is selected among mood stabilizers such as lithium or its salts including lithium carbonate, lithium citrate, lithium sulfate, lithium orotate, and lithium oxybutyrate; or anticonvulsants such as valproic acid, divalproex sodium, lamotrigine, carbamazepine, gabapentin, topiramate and oxcarbazepine. More preferably, said further agent is lithium or one of its salts. The physician would be able to adapt and optimize appropriate medical care of a patient, by carefully selecting the appropriate further agent according the invention. This choice would depend, *inter alia,* of the initial diagnosis of the patient and the severity of the affection and the experienced symptoms.

The compound of the invention and the further agent may be combined with pharmaceutically acceptable excipients, and optionally sustained-release matrices, such as biodegradable polymers, to form pharmaceutical compositions. Therefore, **in a third aspect,** the invention relates to a pharmaceutical composition.

**"Pharmaceutically"** or "**pharmaceutically acceptable**" refers to molecular entities and compositions that do not produce an adverse, allergic or other untoward reaction when administered to a mammal, especially a human, as appropriate. A pharmaceutically acceptable carrier or excipient refers to a non-toxic solid, semisolid or liquid filler, diluent, encapsulating material or formulation auxiliary of any type.

The form of the pharmaceutical compositions, the route of administration, the dosage and the regimen naturally depend upon the condition to be treated, the severity of the illness, the age, weight, and sex of the patient, etc.

The pharmaceutical compositions of the invention can be formulated for a topical, oral, intranasal, intraocular, intravenous, intramuscular or subcutaneous administration and the like. Preferably, the pharmaceutical compositions of the invention can be formulated for an oral administration.

The pharmaceutical compositions can take the form of tablets, pills, capsules, semisolids, powders, sustained release formulations, solutions, suspensions, emulsions, syrups, elixirs, aerosols, or any other appropriate compositions; and comprise at least one compound according this invention.

Because of their ease in administration, tablets and capsules represent the most advantageous oral dosage unit form, in which case solid pharmaceutical carriers are obviously employed. If desired, tablets may be sugar coated or enteric coated by standard techniques. The tablets or pills can be coated to provide a dosage form affording the advantage of prolonged action. For example, the tablet or pills can comprise an inner dosage and an outer dosage component, the latter being in the form of an envelope over the former. The two components can be separated by an enteric layer, which serves to resist disintegration in the stomach and permits the inner component to pass intact into the duodenum or to be delayed in release. A variety of material can be used for such enteric layers or coatings, such materials including a number of polymeric acids with such materials as shellac, cetyl alcohol and cellulose acetate.

The compound of the invention and the further agent may also be administered in the form of liposome delivery systems, such as small unilamellar vesicles, large unilamellar vesicles and multilamellar vesicles. Liposomes can be formed from a variety of phospholipids, such as cholesterol, stearylamine or phosphatidylcholines.

Preferably, the pharmaceutical compositions contain vehicles which are pharmaceutically acceptable for a formulation capable of being injected. These may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions.

The pharmaceutical forms suitable for injectable use include sterile aqueous solutions or dispersions; formulations including sesame oil, peanut oil or aqueous propylene glycol; and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms, such as bacteria and fungi. Solutions of the active compounds as free base or pharmacologically acceptable salts can be prepared in water suitably mixed with a surfactant, such as hydroxypropylcellulose. Dispersions can also be prepared in glycerol, liquid polyethylene glycols, mixtures thereof and in oils. Under ordinary conditions of storage and use, these preparations contain a preservative to prevent the growth of microorganisms.

The carrier can also be a solvent or dispersion medium containing, for example, water, ethanol, polyol (for example, glycerol, propylene glycol, and liquid polyethylene glycol, and the like), suitable mixtures thereof, and vegetables oils. The proper fluidity can be maintained, for example, by the use of a coating, such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for

example, sugars or sodium chloride. Prolonged absorption of the injectable compositions can be brought about by the use in the compositions of agents delaying absorption, for example, aluminium monostearate and gelatin.

Sterile injectable solutions are prepared by incorporating the active compounds in the required amount in the appropriate solvent with various of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof. Upon formulation, solutions will be administered in a manner compatible with the dosage formulation and in such amount as is therapeutically effective. The formulations are easily administered in a variety of dosage forms, such as the type of injectable solutions described above, but drug release capsules and the like can also be employed.

For parenteral administration in an aqueous solution, for example, the solution may be suitably buffered and the liquid diluent first rendered isotonic with sufficient saline or glucose. These particular aqueous solutions are especially suitable for intravenous, intramuscular, subcutaneous and intraperitoneal administration. In this connection, sterile aqueous media which can be employed will be known to those of skill in the art in light of the present disclosure. For example, one dosage could be dissolved in 1 ml of isotonic NaCl solution and either added to 1000 ml of hypodermoclysis fluid or injected at the proposed site of infusion, (see for example, "Remington's Pharmaceutical Sciences" 15th Edition, pages 1035-1038 and 1570-1580). Some variation in dosage will necessarily occur depending on the condition of the subject being treated. The person responsible for administration will, in any event, determine the appropriate dose for the individual subject.

For aerosol administration, the compound of the invention and the further agent are preferably supplied in finely divided from along with a surfactant and propellant. The surfactant must, of course, be nontoxic, and preferably soluble in the propellant. Representative of such agents are the esters or partial esters of fatty acids containing from 6 to 22 carbon atoms, such as caproic, octanoic lauric, palmitic, stearic, linoleic, linolenic, olesteric and oleic acids with an aliphatic polyhydric alcohol or its cyclic anhydride. Mixed esters, such as mixed or natural glycerides may be employed. A carrier can also be included, as desired, as with, e.g., lecithin for intranasal delivery. An example includes a solution in which each milliliter included 7.5 mg NaCl, 1.7 mg citric acid monohydrate, 3 mg disodium phosphate dihydrate and 0.2 mg benzalkonium chloride solution (50%) (Gozes et al., J Mol Neurosci. 19(1-2):167-70 (2002)).

The doses used for the administration can be adapted as a function of various parameters, and in particular as a function of the mode of administration used, of the relevant pathology, or alternatively of the desired duration of treatment. It will be appreciated that appropriate dosages of the compounds, and compositions comprising the compounds, can vary from patient to patient. Determining the optimal dosage will generally involve the balancing of the level of therapeutic benefit against any risk or deleterious side effects of the treatments described herein. The selected dosage level will depend on a variety of factors including, but not limited to, the activity of the particular compound, the route of administration, the time of administration, the rate of excretion of the compound, the duration of the treatment, other drugs, compounds, and/or materials used in combination, and the age, sex, weight, condition, general health, and prior medical history of the patient. The amount of compound and route of administration will ultimately be at the discretion of the physician, although generally the dosage will be to achieve local concentrations at the site of action which achieve the desired effect without causing substantial harmful or deleterious side-effects.

**In a fourth aspect,** the invention relates to said pharmaceutical composition for use in therapy, preferably for treating bipolar and related disorders.

In **a fifth aspect,** the invention relates to at least one compound of formula (I), and at least one further agent as a combined preparation for simultaneous, separate or sequential use for treating of bipolar or related disorders, wherein said further agent is selected from the group consisting:

- mood stabilizers such as lithium or its salts including lithium carbonate, lithium citrate, lithium sulfate, lithium orotate, and lithium oxybutyrate;
- anticonvulsants such as valproic acid, divalproex sodium, lamotrigine, carbamazepine, gabapentin, topiramate and oxcarbazepine;
- atypical antipsychotics such as olanzapine, aripiprazole, quetiapine, risperidone, ziprasidone, asenapine, paliperidone, and lurasidone.

All the previously disclosed technical data are applicable here.

***Methods of use***

The methods described herein include methods of treating a bipolar disorder or a related disorder in a subject

in need of treatment, comprising administering to the subject a therapeutically effective amount of at least one compound of formula (I) as defined herein.

All the previously technical data disclosed herein are applicable to the methods of the invention.

The invention will be further illustrated by the following examples. However, these examples should not be interpreted in any way as limiting the scope of the present invention.

## FIGURES LEGENDS

**Figure 1 :** Dose-response curves of cariporide (CARI), zoniporide (ZONI) eniporide (ENI), dimethyl-amiloride (DMA) and hexamethyl-amiloride (HMA) according to example 1.

**Figure 2:** Histogram representing the % of endocytosis at different concentrations of zoniporide according to example 2.

## EXAMPLES

All starting materials were commercially available and used without further purification. They were purchased from Sigma Aldrich or Cayman Chemicals.

### Example 1: Pharmacological Characterization of NHE7 inhibitors:

The PM-NHE7 cells selected for NHE7 expression at the plasma membrane (Milosavljevic N, Monet M, Léna I, Brau F, Lacas-Gervais S, Feliciangeli S, Counillon L, Poet M., The intracellular Na(+)/H(+) exchanger NHE7 effects a Na(+)-coupled, but not K(+)-coupled proton-loading mechanism in endocytosis. Cell rep. 7, 1-8, May 8, 2014, 689-696) were seeded to confluence on 24-well plates and incubated for 60 minutes at 37°C in a medium containing 50 mM $NH_4Cl$ , 70mM Choline Chloride, 2 mM $CaCl_2$, 1 mM $MgCl_2$, 5 mM glucose, 15 mM Hepes (pH 7.4). The cells were then rinsed twice in less than 15 seconds with 120 mM choline chloride buffered to pH 7.4 with 15 mM Tris, 1 mM $MgCl_2$, 2 mM $CaCl_2$. The uptake of lithium was then started by adding the same medium containing 3 to 10 mM LiCl and the various inhibitor concentrations. Under these conditions, Lithium uptake is linear. All dose-response curves for the inhibitors tested in this study were therefore measured under initial velocity conditions in which equation (1) applies. At the end of the uptake time the cells were rinsed rapidly (4 rinses in less than 10 seconds) in PBS at 4° C. 250μl of 25% nitric acid were added per well. The cell contents were then transferred into eppendorf tubes. The tubes were centrifuged for 5 min at 13000 rpm at room temperature. The supernatant is used to measure the activity of NHE7 (or NHE1 in control) by measuring intracellular the $Li^+$ concentrations (which are due to NHE7 transport) by atomic absorption spectroscopy. (Thermo ICE 3000 Series AA Spectrometer).

Data obtained from the uptake measurements were used to plot the dose responses curves i.e. the % of maximal lithium uptake as a function of the inhibitors concentrations (Log scale, figure 1). This figure shows that eniporide and zoniporide have very good affinity with NHE7.

Because acylguanidines are competitive inhibitors of NHE7, the dose response curves of different concentrations of inhibitors on the rate of transport are given by equation (1) below:

$$V = Vmax\,[Li^+]/(Km_{app} + [Li^+])$$
$$\text{With } Km_{app} = Km\,(1+[I]/Ki) \qquad (1)$$

- where [I] is the inhibitor concentration,
- Vmax is the maximum speed of the exchanger, expressed in (100)% in the dose response curves,
- Km is the value of the Michaelis constant for Lithium (see Milosavljevic et al., Cell rep. 7, 1-8, May 8, 2014, 689-696 for NHE7)
- Ki the dissociation constant for the inhibitor under consideration.

It is simple to obtain a linear equation by plotting the Maximal rate (w/o inhibitor) versus the rates in the presence of inhibitor, as a function of the inhibitor concentration. The slope is then proportional to 1/Ki. The calculated Ki are reported in table 1.

**Table 1: Ki of the tested inhibitors**

| Km (mM) or Ki (μM) | NHE7 | NHE1 |
|---|---|---|
| $Na^+$ (Km) | 200 | 30 |
| $Li^+$ (Km) | 16 | 10 |
| Amiloride (Ki) Compound (II) | 1.64 | 3.27 |
| EIPA (Ki) Compound (VI) | 0.04 | 0.1 |
| DMA (Ki) Compound (III) | 0.17 | 0.1 |
| Cariporide (Ki) Compound (VII) | 0.02 | 0.04 |
| HMA (Ki) Compound (IV) | 0.08 | 0.02 |
| Eniporide (Ki) Compound (VIII) | 0.0025 | 0.04 |
| Zoniporide (Ki) Compound (IX) | 0.003 | 0.02 |

All the tested inhibitors show a better affinity to NHE7 than lithium. In particular eniporide and zoniporide have a very good affinity to NHE7. Moreover, this affinity is selective to NHE7 compare to NHE1.

**Example 2: Endocytosis kinetics**

200 mg/ml Alexa 555 transferrin (Invitrogen) was bound for various times at 37°C on WT NHE7 transfected cells (that express NHE7 specifically in intracellular compartments) in control conditions or in the presence of zoniporide at the defined concentrations. After five minutes of incubation, the coverslips were rapidly rinsed in ice-cold PBS and the cells were fixed in ice-cold 4% PFA and then permeabilized with 2% saponin. The nuclei were then stained with DAPI. These different steps rinse the transferrin, which has not been internalized, and stop intracellular traffic. This makes it possible to visualize the endocytosis at different times. The coverslips were then mounted and photographed for image analysis and quantification. Images were taken using confocal (LSM780, Carl Zeiss) or super resolution microscopy coupled to image deconvolution (Deltavision Elite Imaging System).
Endocytosis was quantified as the normalized fluorescence accumulation in intracellular clusters as a function of time using a home-made macro (Mica Imaging platform, Nice) on Image J NIH ImageJ (Schneider, C. A.; Rasband, W. S. & Eliceiri, K. W. (2012), "NIH Image to ImageJ: 25 years of image analysis", Nature methods 9(7): 671-675). Briefly, the macro first identifies the DAPI blue-colored cell nuclei and then traces a mask located at a fixed distance from the edge of the nucleus, thus corresponding to the inside of the cell to which the nucleus belongs. Then, all the clusters of red pixels (Alexa transferrin 555) with a density, a shape and a surface defined by the macro (and parameterizable by the user) are quantified inside each mask. The data (surface, number and intensity) were then directly saved in Microsoft Excel format for compilation and statistical analysis.
Histograms were compiled from at least five independent experiments, in which experimental points were at least duplicates. Data are presented as mean values and SEM (error bars). Constants are provided with the error of the fits. The groups were compared by one-way ANOVA. Differences were considered significant if $p < 0.05$ (*) and $p < 0.01$ (**).
The data obtained are presented in figure 2. These results show the dose-dependent effect of zoniporide on the endocytosis.

**Claims**

**1.** Compound for use in treating bipolar and related disorders, wherein said compound is represented by formula (I):

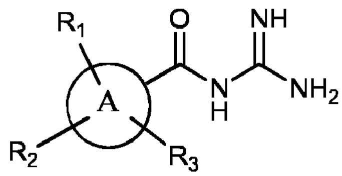

(I)

wherein :

- A is a cycle selected from the group consisting of phenyl and heteroaryl having 5 or 6 ring atoms;
- $R_1$ is selected from the group consisting of $C_1$-$C_6$ alkyl, $C_3$-$C_6$ cycloalkyl, $C_1$-$C_6$ haloalkyl, halogen, $C_1$-$C_6$ alkylsulfonyl, $C_1$-$C_6$ alkylsulfoxide, heteroaryl having 5 to 10 ring atoms, aryl having 6 to 10 ring atoms, heterocycle having 5 to 7 ring atoms, $-NR_4R_5$, $-OR_6$, nitro, cyano, sulfonate, and aminosulfonyl ;

  - $R_4$, $R_5$ and $R_6$ being independently chosen among hydrogen and $C_1$-$C_6$ alkyl;

- $R_2$ is selected from the group consisting of $C_1$-$C_6$ alkyl, $-NR_4R_5$, $-OR_6$, $C_7$-$C_{16}$ arylalkyl, $C_7$-$C_{16}$ alkylaryl, $C_1$-$C_6$ alkenyl, and $C_1$-$C_6$ alkynyl, heteroaryl having 5 to 10 ring atoms, aryl having 6 to 10 ring atoms and heterocycle having 5 to 7 ring atoms, said heterocycle being optionally substituted by - (C=O)-$R_7$;

  - $R_4$, $R_5$ and $R_6$ being as defined above,
  - $R_7$ being chosen among heteroaryl having 5 to 10 ring atoms and aryl having 6 to 10 ring atoms;

- $R_3$ can be present or absent and, if present, $R_3$ is selected from the group consisting of $C_1$-$C_6$ alkyl, and $-NR_4R_5$,

  - $R_4$ and $R_5$ being as defined above;

and wherein any alkyl, cycloalkyl, haloalkyl, alkylsulfonyl, alkylsulfoxide, heteroaryl, aryl, heterocycle, arylalkyl, alkylaryl, alkenyl or alkynyl group is optionally substituted with one or more substituents selected from the group consisting of $C_1$-$C_6$ alkyl, halogen, $C_1$-$C_6$ haloalkyl, oxo, nitro, cyano, $-NH_2$, -OH, - SH, -COOH, and $-CONH_2$.

**2.** Compound for use according to claim 1, wherein

- A is a cycle selected from the group consisting of phenyl and heteroaryl having 5 or 6 ring atoms;
- $R_1$ is selected from the group consisting of $C_1$-$C_6$ alkyl, $C_3$-$C_6$ cycloalkyl, $C_1$-$C_6$ haloalkyl, halogen, $C_1$-$C_6$ alkylsulfonyl, heteroaryl having 5 to 10 ring atoms, aryl having 6 to 10 ring atoms, heterocycle having 5 to 7 ring atoms, and $-NR_4R_5$;

  - $R_4$ and $R_5$ being independently chosen among hydrogen and $C_1$-$C_6$ alkyl;

- $R_2$ is selected from the group consisting of $C_1$-$C_6$ alkyl, $-NR_4R_5$, heteroaryl having 5 to 10 ring atoms, aryl having 6 to 10 ring atoms, and heterocycle having 5 to 7 ring atoms, said heterocycle being optionally substituted by - (C=O)-$R_7$,;

  - $R_4$ and $R_5$ being as defined above,
  - $R_7$ being chosen among heteroaryl having 5 to 10 ring atoms and aryl having 6 to 10 ring atoms;

- $R_3$ can be present or absent and, if present, $R_3$ is selected from the group consisting of $C_1$-$C_6$ alkyl, and $-NR_4R_5$,

  - $R_4$ and $R_5$ being as defined above.

**3.** Compound for use according to claim **1 or 2,** wherein A is selected from the group consisting of

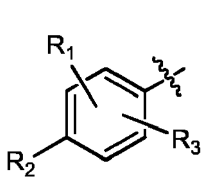, 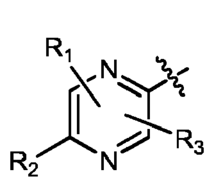, 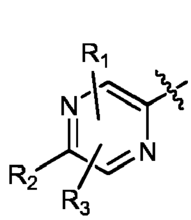, and 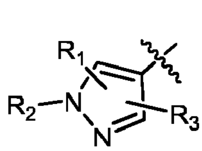.

**4.** Compound for use according to any of the preceding claims, wherein A is selected from the group consisting of

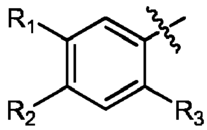, 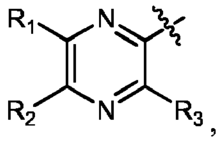, 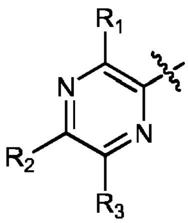, and 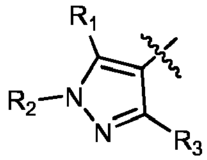.

5. Compound for use according to any of the preceding claims, wherein

- $R_1$ is selected from the group consisting of $C_3$-$C_6$ cycloalkyl, halogen, $C_1$-$C_6$ alkylsulfonyl, and $C_1$-$C_6$ haloalkyl;
- $R_2$ is selected from the group consisting of heteroaryl having 5 to 10 ring atoms and aryl having 6 to 10 ring atoms; and,
- $R_3$ can be present or absent and, if present, $R_3$ is a $C_1$-$C_6$ alkyl group.

6. Compound for use according to any of the preceding claims, wherein said compound is selected in the group consisting of the compounds as represented by formula (II), formula (III), formula (IV), formula (V), formula (VI), formula (VII), formula (VIII), and formula (IX):

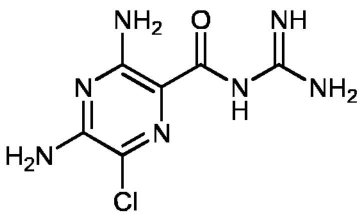 (II) ; 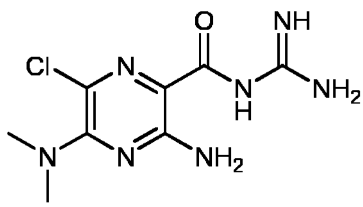 (III)

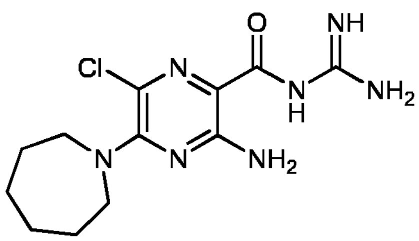 (IV) ; 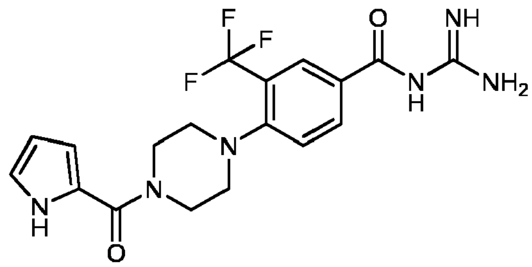 (V)

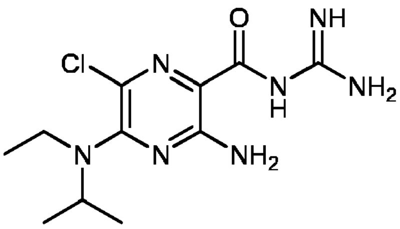 (VI); 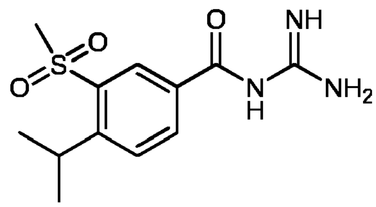 (VII)

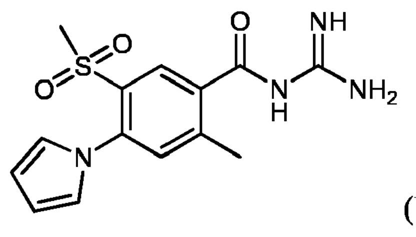 (VIII); 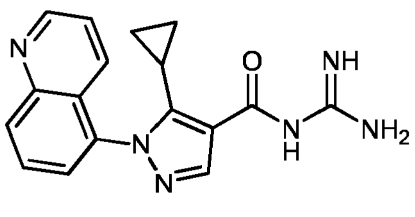 (IX).

7. Compound for use according to claim 6, wherein said compound is selected in the group consisting of the compounds

as represented by formula (VIII) and (IX).

**8.** Compound for use according to any one of claims **1 to 7,** for treating bipolar and related disorders by inhibiting NHE7.

**9.** Compound for use according to any one of claims **1 to 7,** for treating bipolar and related disorders in patients who do not show psychotic symptoms.

**10.** A composition comprising:

(i) at least one compound of formula (I) as defined in any one of claims **1** to **7**; and
(ii) at least one further agent selected from the group consisting:

- mood stabilizers such as lithium or its salts including lithium carbonate, lithium citrate, lithium sulfate, lithium orotate, and lithium oxybutyrate;
- anticonvulsants such as valproic acid, divalproex sodium, lamotrigine, carbamazepine, gabapentin, topiramate and oxcarbazepine; and
- atypical antipsychotics such as olanzapine, aripiprazole, quetiapine, risperidone, ziprasidone, asenapine, paliperidone, and lurasidone.

**11.** The composition of claim **10** for use in therapy.

**12.** The composition of claim **10** for use in treating bipolar and related disorders.

**13.** A compound of formula (I) as defined in any one of claims **1** to **7**, and at least one further agent as a combined preparation for simultaneous, separate or sequential use in the treatment or prophylaxis of bipolar disorder, wherein said agent is selected from the group consisting:

- mood stabilizers such as lithium or its salts including lithium carbonate, lithium citrate, lithium sulfate, lithium orotate, and lithium oxybutyrate;
- anticonvulsants such as valproic acid, divalproex sodium, lamotrigine, carbamazepine, gabapentin, topiramate and oxcarbazepine; and
- atypical antipsychotics such as olanzapine, aripiprazole, quetiapine, risperidone, ziprasidone, asenapine, paliperidone, and lurasidone.

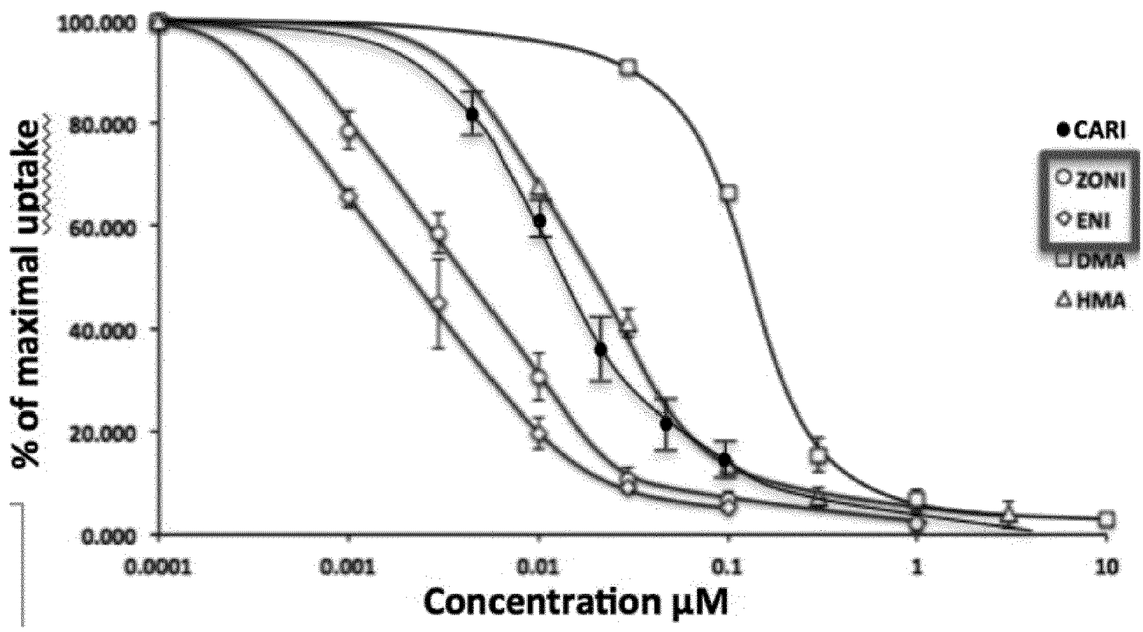


Figure 1

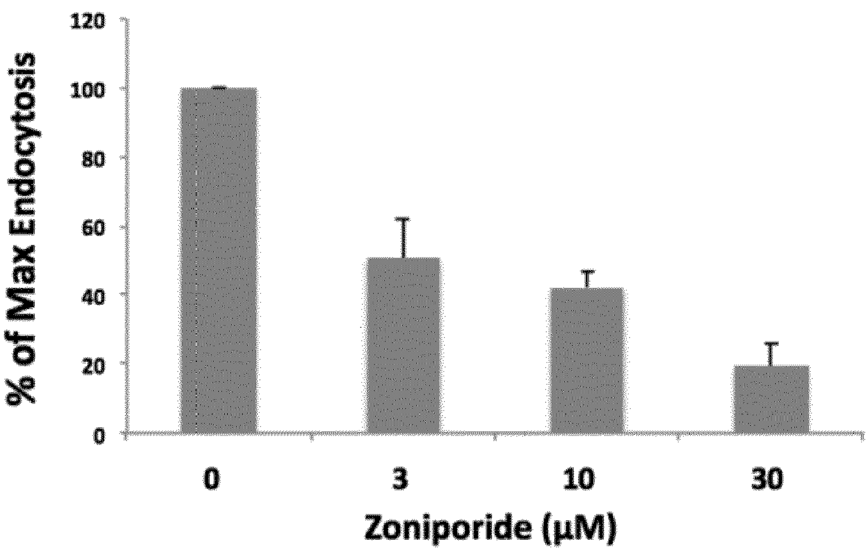


Figure 2

Europäisches Patentamt
European Patent Office
Office européen des brevets

# EUROPEAN SEARCH REPORT

Application Number
EP 17 30 5553

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | MILOSAVLJEVIC ET AL.: "The Intracellular Na+/H+ Exchanger NHE7 Effects a Na+-Coupled, but Not K+-Coupled Proton-Loading Mechanism in Endocytosis", CELL REPORTS, vol. 7, no. 3, 1 May 2014 (2014-05-01), pages 689-696, XP055396565, US ISSN: 2211-1247, DOI: 10.1016/j.celrep.2014.03.054 * Page 694, paragraph bridging left and right columns; page 691, right column, section: "NHE7 inhibition by acylguanidines and vesicular protons". * ----- | 1-13 | INV. A61K31/4965 A61K31/155 A61K31/402 A61K31/4709 A61K31/496 A61K31/55 A61P25/18 |
| X | WO 2013/139700 A1 (AC IMMUNE SA [CH]; UNIV BASEL [CH]) 26 September 2013 (2013-09-26) * Page 14, lines 11-20; page 14, line 29 to page 15, line 1; page 30, lines 19-22; claims 14 and 25. * ----- | 1-13 | |
| X | US 4 894 376 A (MORAD MARTIN [US] ET AL) 16 January 1990 (1990-01-16) * Column 3, line 50 to column 4, line 7. * ----- | 1-13 | TECHNICAL FIELDS SEARCHED (IPC) A61K A61P |

The present search report has been drawn up for all claims

1

| Place of search | Date of completion of the search | Examiner |
|---|---|---|
| Munich | 8 August 2017 | Weisbrod, Thomas |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT ON EUROPEAN PATENT APPLICATION NO.** EP 17 30 5553

This annex lists the patent family members relating to the patent documents cited in the above-mentioned European search report. The members are as contained in the European Patent Office EDP file on
The European Patent Office is in no way liable for these particulars which are merely given for the purpose of information.

08-08-2017

| Patent document cited in search report | Publication date | Patent family member(s) | Publication date |
|---|---|---|---|
| WO 2013139700 A1 | 26-09-2013 | NONE | |
| US 4894376 A | 16-01-1990 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- Diagnostic and Statistical Manual of Mental Disorders
- Remington's Pharmaceutical Sciences. 1035-1038, 1570-1580
- **GOZES et al.** *J Mol Neurosci.,* 2002, vol. 19 (1-2), 167-70
- **MILOSAVLJEVIC N ; MONET M ; LÉNA I ; BRAU F ; LACAS-GERVAIS S ; FELICIANGELI S ; COUNILLON L ; POET M.** The intracellular Na(+)/H(+) exchanger NHE7 effects a Na(+)-coupled, but not K(+)-coupled proton-loading mechanism in endocytosis. *Cell rep.,* 08 May 2014, vol. 7, 1-8
- **MILOSAVLJEVIC et al.** *Cell rep.,* 08 May 2014, vol. 7 (1-8), 689-696
- **SCHNEIDER, C. A. ; RASBAND, W. S. ; ELICEIRI, K. W.** NIH Image to ImageJ: 25 years of image analysis. *Nature methods,* 2012, vol. 9 (7), 671-675